## Europäisches Patentamt
## European Patent Office
## Office européen des brevets

(19)

(11) Veröffentlichungsnummer: **0 170 843 B1**

# EUROPÄISCHE PATENTSCHRIFT

(45) Veröffentlichungstag der Patentschrift: **19.11.92**

(51) Int. Cl.5: **A61K 37/66**, //(A61K37/66, 37:02)

(21) Anmeldenummer: **85107618.2**

(22) Anmeldetag: **20.06.85**

(54) Synergistische Mischungen von Interferonen und Tumor-Nekrose-Faktor.

(30) Priorität: **23.06.84 DE 3423234**

(43) Veröffentlichungstag der Anmeldung:
**12.02.86 Patentblatt 86/07**

(45) Bekanntmachung des Hinweises auf die Patenterteilung:
**19.11.92 Patentblatt 92/47**

(84) Benannte Vertragsstaaten:
**AT BE CH DE FR GB IT LI LU NL SE**

(56) Entgegenhaltungen:
**EP-A- 0 131 789**
**DE-A- 3 227 262**

**CHEMICAL ABSTRACTS, Band 100, Nr. 7, 13. Februar 1984, Seite 441, no. 49841x, Columbus, Ohio, US; J. IMANISHI u.a.: "Production and characterization of human tumor-degenerating factor (TDF)", & C.R. SEANCES SOC BIOL . SIS FIL. 1983, 177(4), 570-3**

(73) Patentinhaber: **BOEHRINGER INGELHEIM INTERNATIONAL GmbH**
**Postfach 20**
**W-6507 Ingelheim am Rhein(DE)**

(72) Erfinder: **Adolf, Günther, Dr.**
**Johannagasse 20/7**
**A-1050 Wien(AT)**

## Beschreibung

Gegenstand der vorliegenden Erfindung sind ternäre Mischungen eines Interferons vom Typ alpha oder beta mit einem Interferon des Typs gamma und Tumor-Nekrose-Faktor, wobei die Bestandteile zu mindestens 90% frei von Verunreinigung sind. Die Mischungen zeigen jeweils höhere cytostatische und/oder cytotoxische Wirkung auf transformierte Zellen, als auf Grund der Aktivität der einzelnen Komponenten oder binärer Gemische davon zu erwarten gewesen wäre.

Mehrere in höheren Organismem natürlich vorkommende Proteine sind in der Lage, die Proliferation von Tumorzellen in vitro und in vivo zu hemmen. Dazu gehören unter anderem Interferone, Lymphotoxine und Tumor-Nekrose-Faktoren.

Interferone werden gegenwärtig in drei Klassen eingeteilt, die sich in ihren biologischen und physikalisch-chemischen Eigenschaften unterscheiden. Alpha-Interferone (IFN-$\alpha$) umfassen im Menschen und einer Reihe von anderen bisher untersuchten Säugetieren eine Familie von untereinander in Aminosäuresequenz und Eigenschaften sehr ähnlichen Proteinen (1,2,3). Beta-Interferone (IFN-$\beta$) werden im Menschen sowie in den meisten anderen untersuchten Säugetierspezies durch ein einziges Protein repräsentiert; in Rindern sind jedoch mehrere IFN-$\beta$ Subtypen vorhanden (4). Humanes IFN-$\beta$ zeigt partielle Sequenzhomologie zu humanem IFN-$\alpha$, ist wie dieses relativ säurestabil und scheint an den selben Membranrezeptor zu binden, kann jedoch zum Beispiel serologisch sowie durch sein Aktivitätsspektrum auf heterologen Zellen von IFN-$\alpha$ unterschieden werden (1). Gamma-Interferon (IFN-$\gamma$), das im Menschen wie in allen anderen bisher untersuchten Spezies durch lediglich ein Gen kodiert wird, ist in seiner Aminosäuresequenz mit IFN-$\alpha$ und IFN-$\beta$ nicht oder nur sehr entfernt verwandt, ist säurelabil und unterscheidet sich auch in anderen Eigenschaften deutlich von IFN-$\alpha$ und IFN-$\beta$ (1,5,6,7).

In der Literatur werden daher IFN-$\alpha$ und IFN-$\beta$ gelegentlich als Typ-I Interferone, IFN-$\gamma$ als Typ-II Interferon bezeichnet. Alle Interferone zeigen antivirale Aktivität und haben eine Reihe von immunmodulatorischen Wirkungen (8). Typ-I- und Typ-II Interferon - Mischungen zeigen in verschiedenen Systemen synergistische Wirkung (9).

In den letzten Jahren konnten durch die Fortentwicklung molekularbiologischer Arbeitsmethoden alle Klassen von menschlichen Interferonen kloniert und in Mikroorganismen produziert werden. Dadurch konnten erstmals hinreichend große Mengen von bis zur Homogenität gereinigten Interferonen hergestellt und für klinische Prüfungen zur Verfügung gestellt werden. Die bisher vorliegenden Berichte über klinische Studien zeigen jedoch, daß bei einer Reihe von malignen Erkrankungen keine oder nur unbefriedigende Therapieerfolge zu verzeichnen sind.

Ferner wurde beobachtet, daß eine Verbesserung des Therapieerfolges durch Erhöhung der applizierten IFN-Dosis auf Grund von untolerierbaren Nebenwirkungen oft nicht möglich ist (10.11.12).

Tumor-Nekrose-Faktor (TNF) wurde ursprünglich als ein in Bacillus Calmette-Guerin sensivierten Versuchstieren durch Endotoxin-Behandlung induzierbares Protein beschrieben (13). Diese Substanz zeigt in vitro cytostatische und/oder cytotoxische Effekte auf eine Reihe von Tumorzellinien und bewirkt in Tiermodellen eine hämorraghische Nekrose gewisser transplantierbarer Tumoren. Vor kurzem wurde berichtet, daß humane B-lymohoblastoide Zellinien spontan ein Protein produzieren, das nach verschiedenen Kriterien als Humaner-Tumor-Nekrose-Faktor (Hu-TNF) zu bezeichnen ist (14).

Partiell gereinigte Kulturüberstände dieser Zellen zeigten synergistische antiproliferative Wirkung mit humanem Interferon-$\alpha$ oder Interferon-$\gamma$ (14). Wie aus der Literatur bekannt ist, produzieren diese Zellen jedoch nicht nur TNF, sondern geben auch eine Reihe anderer Lymphokine ins Medium ab, darunter IFN-$\alpha$, Makrophagen-Aktivierungsfaktor, Migration Inhibitory Factor und Skin Reactive Factor (15, 16). Es kann daher nicht ausgeschlossen werden, daß eines oder mehrere dieser Proteine oder auch andere, bisher nicht charakterisierte Faktoren an den Effekten beteiligt sind.

In der älteren, nach dem Anmeldetag der vorliegenden Erfindung veröffentlichten Europäischen Patentanmeldung EP-A 131 789 werden konkret binäre Mischungen aus hTNF mit IFN-$\alpha$ oder IFN-$\gamma$ beschrieben, welche gegenüber den Einzelkomponenten synergistische Wirkungen zeigen. Zwar werden auch Mischungen, bestehend aus hTNF, IFN-$\alpha$, IFN-$\beta$ und IFN-$\gamma$ bzw. unterschiedliche Kombinationen davon, offenbart, jedoch konkret nicht ternäre Mischungen. Außerdem liegt in den besagten Mischungen zumindest der hTNF in so unreiner Form vor, daß auch andere, unbekannte Lymphokine die beschriebenen Effekte bewirkt haben könnten.

Aufgabe der vorliegenden Erfindung war es daher, die Auswirkung von hochgereinigtem Hu-TNF in einer Mischung mit Interferonen zu untersuchen und Mischungen für die Therapie neoplastischer Erkrankungen des Menschen bereitzustellen.

Gelöst wurde die Aufgabe durch Bereitstellung einer ternären Mischung aus Tumor-Nekrose-Faktor und Interferonen, dadurch gekennzeichnet, daß sie aus Tumor-Nekrose-Faktor, einem Interferon des Typs-I und einem Interferon des Typs-II zusammengesetzt ist, wobei die Bestandteile zu mindestens 90% frei von Verunreinigungen sind.

Erst vor kurzem konnte ein neues Verfahren entwickelt werden, das die Herstellung von Hu-TNF aus Zellen einer permanenten B-lymphoblastoiden Linie in einer Reinheit von mehr als 90% erlaubt (17). Dazu wird die humane B-lymphoblastoide Zellinie RPMI-1788/EBI, hinterlegt bei der CNCM Paris unter der Hinterlegungsnummer I-305 am 23. Mai 1984, in einem geeigneten, serumhaltigen Medium kultiviert, nach anfänglicher Proliferation in serumfreies Medium überführt und mit einem geeigneten Tumorpromotor, beispielsweise Mezerein stimuliert. Um die geforderte hohe Reinheit zu erzielen, wird der Hu-TNF durch porenkontrolliertes Glas (CPG , durch Anionenaustausch- und Lectin-Affinitätschromatographie gereinigt.

Dabei können aus einem Liter Zellkultur etwa 0,1 bis 0,2 mg gereinigter Hu-TNF erhalten werden, sodaß erstmals eine genaue Charakterisierung der biologischen Eigenschaften dieses Proteins möglich wurde, und dessen Auswirkungen in einer Mischung mit Interferonen untersucht werden konnten.

Die antiproliferative (cytostatische/cytotoxische) Wirkung der zu untersuchenden Substanzen wurde wie folgt bestimmt: transformierte Zellen humanen und murinen Ursprungs wurden in Gegenwart von Typ-I-IFN oder Typ-II-IFN der jeweiligen Spezies, von Hu-TNF sowie von Kombinationen dieser Substanzen gezüchtet. Nach fünf bis sechs Tagen wurden die Zellzahlen in den Kulturen bestimmt und mit der Zellzahl unbehandelter Kontrollkulturen verglichen.

Dabei zeigte sich, daß eine Kombination von hochgereinigtem Hu-TNF mit Typ-I-IFN und Typ-II-IFN eine höhere cytostatische und/oder cytotoxische Wirkung aufweist als aus der Addition der Wirkung der Einzelkomponenten zu erwarten gewesen wäre.

Überraschend wurde außerdem festgestellt, daß eine Mischung aller drei Substanzen eine höhere cytostatische und/oder cytotoxische Wirkung hat, als dies auf Grund der Wirkung der drei binären Mischungen vorausgesehen werden konnte.

Diese Ergebnisse zeigen einerseits, daß mit den beschriebenen Mischungen in vitro "therapeutische" Effekte erzielt werden können, die sonst nur mit extrem hohen, untolerierbaren Dosen der Einzelkomponenten erreichbar sind. Darüber hinaus können mit den Mischungen Wirkungen erzielt werden, die selbst mit hohen losen der Einzelkomponenten nicht erzielbar sind.

Diese synergistischen Wirkungen wurden mit hochgereinigten Human-Interferonen (Reinheit≧ 98%), die aus genmanipulierten Bakterien gewonnen worden waren (6 19), mit hochgereinigtem Maus-Interferon- γ aus genmanipulierten Bakterien (Reinheit≧ 95%) (7) sowie mit hochgereinigtem Hu-TNF (Reinheit≧ 90%) (17) erzielt.

Die beobachteten Effekte sind daher als charakteristisch für die untersuchten Interferone bzw. für den Hu-TNF und nicht durch in den Präparaten enthaltene Verunreinigungen verursacht.

Mischungen von humanen-Interferonen und Hu-TNF sind daher für die Therapie neoplastischer Erkrankungen des Menschen von großer Bedeutung.

Vor kurzem konnten für zwei menschliche cytotoxische Proteine die cDNA's in E. coli kloniert werden (25,26). Die aus den DNA-Sequenzen abgeleiteten Aminosäuresequenzen zeigen, daß eines dieser Proteine identisch mit dem ist, das bei der vorliegenden Erfindung verwendet wurde. Beide Proteine haben sehr ähnliche biologische Eigenschaften und ihre Aminosäuresequenzen zeigen einen sehr hohen Homologiegrad, ähnlich denen zwischen IFN-$\alpha$ und IFN-$\beta$ (26).

Es ist daher zu erwarten, daß die in der vorliegenden Erfindung gezeigten synergistischen Wirkungen mit den Interferonen mit beiden Proteinen erzielt werden können.

Im folgenden wird die Erfindung anhand von Beispielen näher erläutert, ohne sie in irgendeiner Weise zu beschränken; insbesondere sei darauf hingewiesen, daß prinzipiell jedes Interferon und jeder TNF, also auch Präparate, die durch andere Verfahren hergestellt werden, impliziert sind.

1. Hemmung der Proliferation der humanen Cervixcarcinom-Zellinie Hela

Zellen der humanen Cervix-Carcinomlinie Hela (21) wurden in Eagle's Minimum Essential Medium (18) in Gegenwart von 10% fötalem Kalbsserum, Penicillin (100 Einheiten/ml) und Streptomycin (50 Einheiten/ml) bei 37 C in einer wasserdampf-gesättigten Atmosphäre von 5% $CO_2$ in Luft gezüchtet. In 3 cm-Kunststoffgewebekulturschalen wurden je 50 000 Zellen in 2.5 ml Medium angesetzt; jeweils 2 Schalen erhielten Zusätze von Interferonen, TNF oder Kombinationen dieser Substanzen in 0.5 ml Medium. Zwei Schalen erhielten nur Medium; diese Kulturen wurden als Referenz für das Wachstum unbehandelter Zellen gewertet. Die Schalen wurden sechs Tage lang wie oben beschrieben inkubiert, anschließend wurden die Zellen mit Hilfe von Trypsin vom Substrat gelöst und die Zahl lebensfähiger Zellen in Hämocytometerkammern bestimmt.

Folgende Interferon- bzw. TNF-Präparate wurden verwendet:

a) Humanes IFN-alpha2arg ("IFN-alpha"), produziert in E. coli (19), Reinheit mindestens 98%, spezifische Aktivität 300x10$^6$ IE/mg (bezogen auf den Referenzstandard G0-23-901-527 der National Institutes of Health, USA), hergestellt von Boehringer Ingelheim, Ingelheim, BRD. Dieses Protein ist identisch mit dem von Streuli et al. (20) beschriebenen Interferon mit Ausnahme der Aminosäure an Position 34 (Arginin anstelle von Histidin).

b) Humanes IFN-gamma, produziert in E. coli (6), Reinheit mindestens 98%, spezifische Aktivität $68 \times 10^6$ E/mg (getestet mit A549-Zellen und Maus-Enzephalomyocarditis- Virus), hergestellt von Genentech Inc., San Francisco, USA.

c) Humaner TNF, Reinheit mindestens 90%, hergestellt wie beschrieben (17).

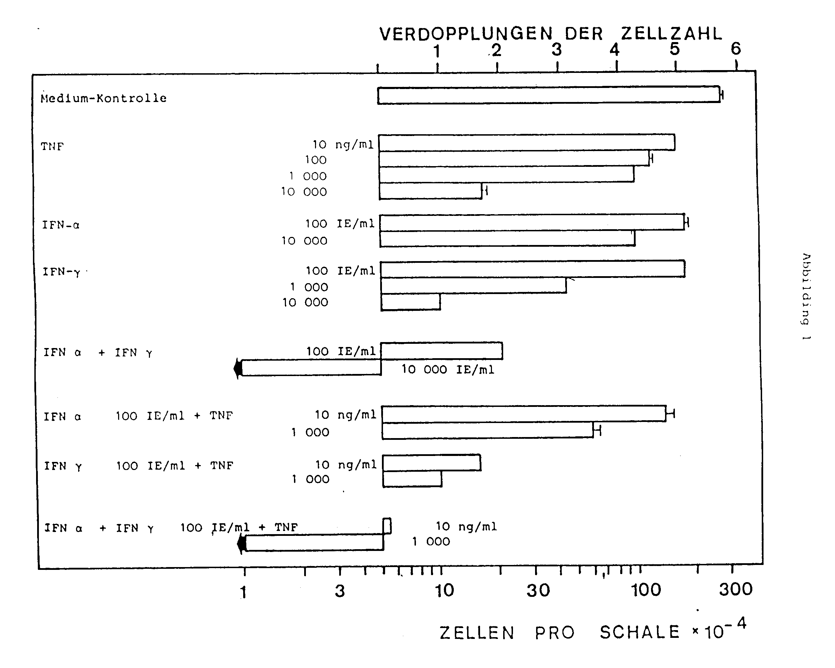

Abbildung 1 zeigt die Ergebnisse des Versuchs. Dargestellt sind die geometrischen Mittelwerte der Zellzahlen in beiden parallel geführten Kulturen. Balken stellen jeweils den höheren der beiden Einzelwerte dar. Pfeile in der Abbildung deuten an, daß weniger als 10 000 Zellen pro Kulturschale gezählt wurden.
Eine Kombination von IFN-gamma (100 E/ml) mit TNF (10 oder 1000 ng/ml) zeigte deutlich höhere Aktivität als auf Grund der Aktivität der Komponenten zu erwarten war; eine Mischung von IFN-alpha, IFN-gamma (jeweils 100 E/ml) und TNF (10 oder 1000 ng/ml) zeigte deutlich höhere Aktivität als dies auf Grund der Aktivität der drei binären Mischungen zu erwarten war.

### 2. Hemmung der Proliferation der humanen Lungencarcinom-Zellinie A549

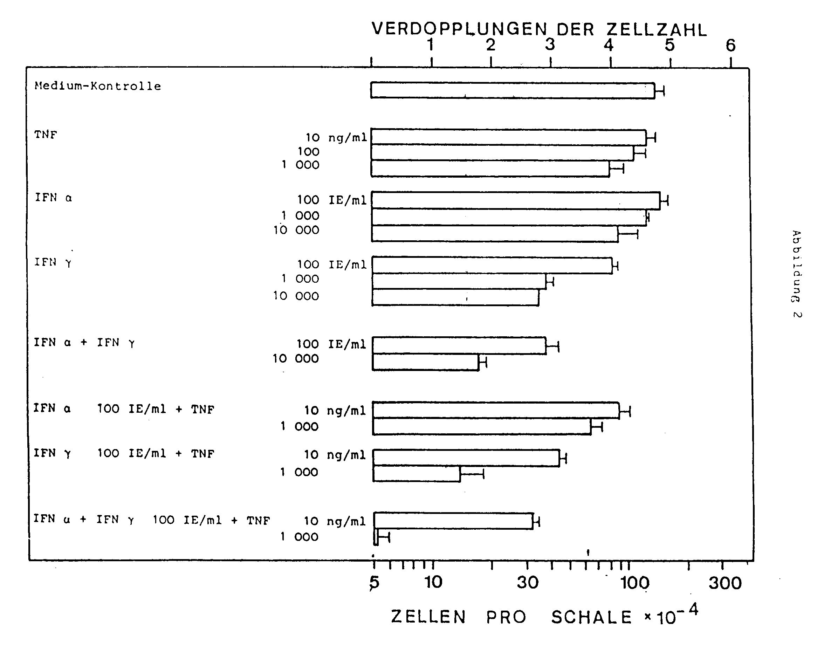

Kultur von Zellen der humanen Lungencarcinom-Zellinie A549 (22) und Behandlung mit Interferonen und TNF wurden wie in Beispiel 1 beschrieben durchgeführt. Die Ergebnisse zeigt Abbildung 2.

Eine Kombination von IFN-gamma (100 E/ml) mit TNF (10 oder 1000 ng/ml) zeigte deutlich höhere Aktivität , als dies auf Grund der Aktivität der Einzelkomponenten zu erwarten war. Eine Kombination von IFN-alpha mit IFN-gamma (jeweils 100 E/ml) und TNF (1000 ng/ml) zeigte höhere Aktivität als dies auf Grund der Wirkung der drei binären Mischungen zu erwarten war.

### 3. Hemmung der Proliferation der Mauszellinie L929

Kultur der Zellen der humanen Linie L929 (23) wurde, wie in Beispiel 1 beschrieben, durchgeführt. Zur Untersuchung der Wachstumshemmung durch Interferone und TNF wurden folgende Präparationen verwendet:

a) Maus-Interferon-alpha/beta, spezifische Aktivität $44 \times 10^6$ E/mg, produziert von Enzo Biochem Inc., New York, USA.

b) Maus-Interferon-gamma, produziert in E. coli (7), Reinheit mindestens 95%, spezifische Aktivität $7 \times 10^6$ E/mg ( getestet auf L929-Zellen mit Enzephalomyocarditis-Virus), hergestellt von Genentech Inc., San Francisco, USA.

c) Humaner TNF, Reinheit mindestens 90%, hergestellt wie beschrieben (17).

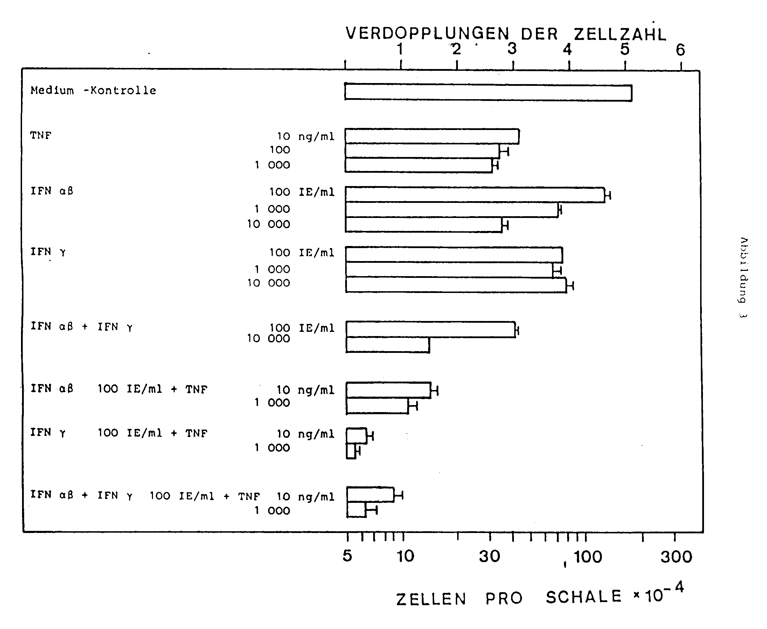

Die Behandlung der Zellen wurde wie in Beispiel 1 beschrieben durchgeführt, die Zellzahl wurde nach fünf Tagen bestimmt. Abbildung 3 zeigt die Ergebnisse.

Eine Behandlung der Zellen mit einer Mischung von IFN-alpha/beta (100 E/ml) und TNF (10 oder 1000 ng/ml) oder IFN-gamma (100 E/ml) und TNF (10 oder 1000 ng/ml) zeigte eine stärkere Wachstumshemmung als dies auf Grund der Wirkung der Komponenten zu erwarten war.

### 4. Hemmung der Proliferation der Maus-Melanomzellinie B16

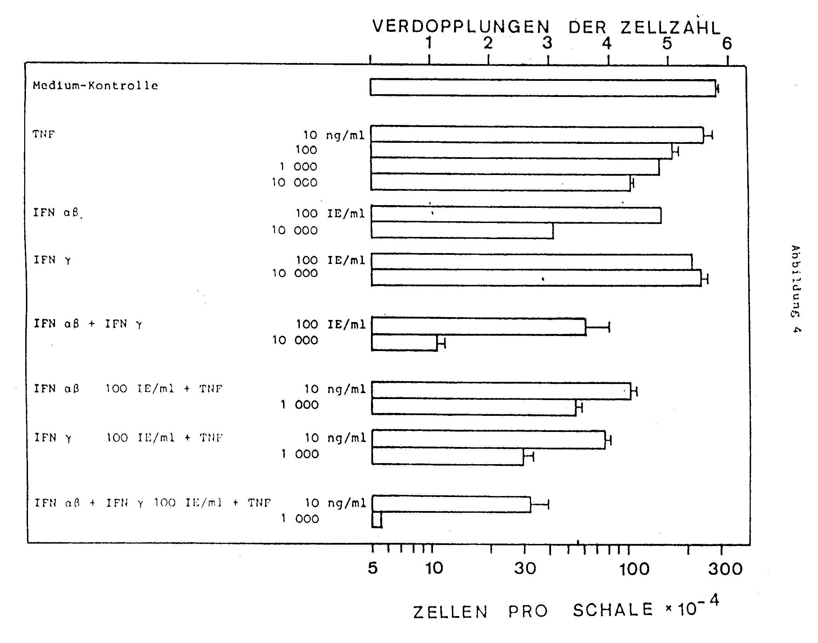

Kultur der Maus-Melanomzellen (24) und Behandlung mit Maus-Interferonen und TNF wurden wie in Beispiel 1 bzw. Beispiel 3 durchgeführt. Die Ergebnisse zeigt Abbildung 4.

Behandlung mit einer Kombination von IFN-alpha/beta (100 E/ml) und TNF (1000 ng/ml) oder einer Kombination von IFN-gamma (100 E/ml) und TNF (10 oder 1000 ng/ml) zeigten höhere Wirkung, als dies auf Grund der Aktivität der Einzelkomponenten zu erwarten war. Eine Kombination von IFN-alpha/beta (100 E/ml) mit IFN-gamma (100 E/ml) und TNF (10 oder 1000 ng/ml) zeigte höhere Aktivität als dies auf Grund der Wirkung der drei binären Kombinationen zu erwarten war.

Literatur

1. Stewart, W.E. (1981)
The interferon system. Springer Verlag, Wien-New York
2. Weissmann, C. (1981)
In: Interferon 1981 (Gresser, I., ed.), pp 101-134, Academic Press, London
3. Weck, P.K., Apperson, S., May, L., & Stebbing, N. (1981)
J. Gen. Virol. 57, 233-237
4. Wilson, V., Jeffreys, A.J., Barrie, P.A., Boseley, P.G., Slocombe, P.M., Easton, A., & Burke, D.C. (1983) J. Mol. Biol. 166, 457-475
5. Epstein, L. (1981)
In: Interferon 1981 (Gresser, I., ed.), pp 13-44 Academic Press, London
6. Gray, P.W., Leung, D.W., Pennica, D.,Yelverton, E., Najarian,R., Simonson, C.C., Derynck, R., Sherwood, P., Wallace, D.M., Berger, S.L., Levinson, A.D. & Goeddel, D.V. (1982) Nature 295, 503-508
7. Gray, P.W. & Goeddel, D.V. (1983)
Proc. Natl. Acad. Sci. USA 80, 5842-5846
8. Friedman, R.M. & Vogel, S.N. (1983)
In: Advances in Immunology, Vol. 34, pp 97-140;

Academic Press, London

9. Czarniecky, C.W., Fennie, C.W., Powers, D.B., & Estell, D.A. J. Virol. 49, 490-496

10. Sikora, K. & Smedley, H. (1983)
Br. Med. J. 286, 739-740

11. Jones, D.H., Bleehen, N.M., Slater, A.J., George, P.J.M., Walker, J.R., & Dixon, A.K. (1983)
Br. J. Cancer 47, 361-366

12. Garna, G., Figlin, R., & Callaghan, M. (1983)
J. Biol. Resp. Modif. 2, 343-347

13. Carswell, E.A., Old, L.J., Kassel, R.L., Green, S., Piore,N. & Williamson, B. (1975)
Proc. Natl. Acad. Sci. USA 72, 3666-3670

14. Williamson, B., Carswell, E., Rubin, B.Y., Prendergast,J. & Old, L.J. (1983)
Proc. Natl. Acad. Sci. USA 80, 5397-5401

15. Schook, L.B., Otz, U., Lazary, S., DeWeck, A., Minowada, J., Odavic, R., Kniep, E.M., & Edy, V. (1981)
In: Lymphokines: A forum for immunoregulatory cell products (Pick, E. & Landy, M., eds) Vol.2, pp 1-19, Academic Press, New York

16. McEntire, J.E., Dunn, P.A., Gehrke, C.W., & Papermaster, B.W. (1981)
In: Lymphokines and Thymic Hormones: Their Potential Utilization in Cancer Therapeutics (Goldstein, A.L. & Chirigos, M.A., eds.) pp 109-119
Raven Press, New York

17. Deutsche Patentanmeldung AZ P 34 21 731.2

18. Eagle, H. (1959)
Science 130, 432

19. Dworkin-Rastl, E., Swetly, P., & Dworkin, M.B. (1983)
Gene 21, 237-248

20. Streuli, M., Nagata, S., & Weissmann, C. (1980)
Science 209, 1343-1347

21. Gey, G.O., Coffman, W.D., & Kubicek, M.T. (1952)
Cancer Res. 12, 264

22. Giard, D.J. (1973)
J.Natl. Cancer Inst. 551, 1417-1423

23. Sanford, K.K., Earle, W.R., & Likely, G.D. (1948)
J. Natl. Cancer Inst. 9, 229

24. Fidler, I.J. (1970)
J. Natl. Cancer Inst. 45, 773

25. Cloning and expression of cDNA for human lymphotoxin, a lymphokine with tumor necrosis activity
Gray, P.W., Aggarwal, B.B., Benton, C.V., Bringman, T.S., Henzel, W.J., Jarrett, J.A., Leung D.W., Moffat, B., Ng, P., Svedersky, L.P., Palladino, M.A., Nedwin, G.E. Nature 312, 721-724 (1984)

26. Human tumor necrosis factor: precursor structure, expression and homology to lymphotoxin
Pennica, D., Nedwin, G.E., Hayflick, J.S., Seeburg, P.H., Derynck, R., Palladino, M.A., Kohr, W.J., Aggarwal, B.B. Goeddel, D.V.
Nature 312, 724-729 (1984)

**Patentansprüche**

1. Mischung aus Tumor-Nekrose-Faktor und Interferonen, dadurch gekennzeichnet, daß sie aus Tumor-Nekrose-Faktor, einem Interferon des Typs-I und einem Interferon des Typs-II zusammengesetzt ist, wobei die Bestandteile zu mindestens 90% frei von Verunreinigungen sind.

2. Mischung nach Anspruch 1, dadurch gekennzeichnet, daß sie Tumor-Nekrose-Faktor, Interferon-alpha und Interferon-gamma enthält.

3. Mischung nach Anspruch 1, dadurch gekennzeichnet, daß sie Tumor-Nekrose-Faktor, Interferon-beta und Interferon-gamma enthält.

4. Mischung nach Ansprüchen 1 bis 3, dadurch gekennzeichnet, daß der Tumor-Nekrose-Faktor und die Interferone des Typs-I und des Typs-II humanen Ursprungs sind.

5. Mischung nach einem der Ansprüche 1 bis 4, dadurch gekennzeichnet, daß der Tumor-Nekrose-Faktor und/oder die Interferone gentechnologisch hergestellt sind.

6. Mischung nach einem der Ansprüche 1 bis 5 zur Verwendung als Arzneimittel.

7. Arzneimittel bestehend aus Tumor-Nekrose-Faktor und Interferonen, dadurch gekennzeichnet, daß es neben üblichen Hilfs- und Trägerstoffen eine Mischung gemäß einem der Ansprüche 1 bis 5 enthält.

8. Verwendung einer Mischung nach einem der Ansprüche 1 bis 5 zur Herstellung einer pharmazeutischen Präparation.

**Claims**

1. Mixture of tumour necrosis factor and interferons, characterised in that it is made up of tumour necrosis factor, a type I interferon and a type II interferon, the constituents being at least 90% free from impurities.

2. Mixture according to claim 1, characterized in that it contains tumour necrosis factor, interferon alpha and interferon gamma.

3. Mixture according to claim 1, characterized in that it contains tumour necrosis factor, interferon beta and interferon gamma.

4. Mixture according to claims 1 to 3, characterised in that the tumour necrosis factor and the type I and type II interferons are of human origin.

5. Mixture according to one of claims 1 to 4, characterised in that the tumour necrosis factor and/or the interferons are produced by genetic engineering.

6. Mixture according to claims 1 to 5 for use as pharmaceutical compositions.

7. Pharmaceutical composition consisting of tumour necrosis factor and interferons, characterised in that it contains a mixture according to one of claims 1 to 5, in addition to conventional excipients and carriers.

8. Use of a mixture according to one of claims 1 to 5 in the production of a pharmaceutical preparation.

**Revendications**

1. Mélange de facteur nécrosant des tumeurs et d'interférons, caractérisé en ce qu'il consiste en facteur nécrosant des tumeurs, en un interféron de type I et en un interféron de type II, les constituants étant exempts d'impuretés à raison d'au moins 90%.

2. Mélange selon la revendication 1, caractérisé en ce qu'il contient du facteur nécrosant des tumeurs, de l'interféron alpha et de l'interféron gamma.

3. Mélange selon la revendication 1, caractérisé en ce qu'il contient du facteur nécrosant des tumeurs, de l'interféron bêta et de l'interféron gamma.

4. Mélange selon les revendications 1 à 3, caractérisé en ce que le facteur nécrosant des tumeurs et les interférons de type I et de type II sont d'origine humaine.

5. Mélange selon l'une des revendications 1 à 4, caractérisé en ce que le facteur nécrosant des tumeurs et/ou les interférons sont préparés par génie génétique.

6. Mélange selon l'une des revendications 1 à 5 utilisable comme médicament.

7. Médicament consistant en facteur nécrosant des tumeurs et en interférons, caractérisé en ce qu'il contient un mélange selon l'une des revendications 1 à 5 et des adjuvants et véhicules courants.

8. Utilisation d'un mélange selon l'une des revendications 1 à 5 pour l'obtention d'une préparation pharmaceutique.

Abbilding 1

VERDOPPLUNGEN DER ZELLZAHL

ZELLEN PRO SCHALE $\times 10^{-4}$

300    100    30    10    3    1

Medium-Kontrolle

TNF
10 ng/ml
100
1 000
10 000

IFN-α
100 IE/ml
10 000

IFN-γ
100 IE/ml
1 000
10 000

IFN α + IFN γ
100 IE/ml
10 000 IE/ml

IFN α    100 IE/ml + TNF
10 ng/ml
1 000

IFN γ    100 IE/ml + TNF
10 ng/ml
1 000

IFN α + IFN γ    100 IE/ml + TNF
10 ng/ml
1 000

Abbildung 2

VERDOPPLUNGEN DER ZELLZAHL

Abbildung 3

EP 0 170 843 B1

ZELLEN PRO SCHALE × $10^{-4}$

**VERDOPPLUNGEN DER ZELLZAHL**

Abbildung 4

ZELLEN PRO SCHALE ×$10^{-4}$

EP 0 170 843 B1